# EUROPEAN PATENT SPECIFICATION

(11) **EP 0 750 482 B1**
(45) Date of publication and mention of the grant of the patent: **05.01.2000**
(21) Application number: 95914747.1
(22) Date of filing: 17.03.1995
(51) Int. Cl.: A61F 2/44, A61F 2/46, A61B 17/02

(54) **INTERVERTEBRAL DISK STABILIZING IMPLANT, AND KIT TO FACILITATE INSERTION THEREOF**
IMPLANTAT ZUR STABILISIERUNG VON BANDSCHEIBEN, UND EINE VORRICHTUNG ZUM ERLEICHTERTEN EINSETZEN DES IMPLANTATS
IMPLANT STABILISATEUR DE DISQUES INTERVERTEBRAUX ET DISPOSITIF D'INSERTION ASSOCIE

(30) Priority: 18.03.1994 US 210229
(43) Date of publication of application: 02.01.1997
(73) Proprietor: PISHARODI, Madhavan, Brownsville, TX 78520 (US)
(72) Inventor: PISHARODI, Madhavan, Brownsville, TX 78520 (US)
(74) Representative: Hanna, Peter William Derek
(86) International application number: PCT/US95/03374
(87) International publication number: WO 95/25487

(56) References cited:
- EP-A- 0 042 271
- EP-A- 0 307 241
- WO-A-92/14423
- DE-A- 3 505 567
- US-A- 3 486 505

## Description

The present invention relates to an intervertebral disk stabilizing implant. More specifically, the present invention relates to generally cylindrically shaped disk implants which are expanded in the middle portion which are used for spinal fusion.

If the spine is injured or becomes diseased, surgical intervention involving removal of one or more disks, and fusion of the adjacent vertebrae, may be indicated. The more frequent injuries are in the lower lumbar and in the lower cervical regions.

Treatment of a herniated disk in the neck and in the lumbar region continues to be a challenging field of medicine. The classical treatment for a ruptured disk is diskectomy, i.e., removal of the disk from between the vertebrae. In this process, all or a portion of the intervertebral disk is removed, leaving a defect which continues to bother the patients throughout the rest of their lives. An additional procedure is to replace the disk space with a bone graft, usually bone chips cut from the patient's iliac crest, bringing about fusion of the vertebrae above and below the disk, eliminating the empty space between the vertebrae.

Diskectomy with fusion is not ideal because the replaced bone does not have the function of the cartilaginous tissue of the disk, i.e. no cushioning effect, and has complications because of several factors. First, conventional bone plugs used to pack the disk space do not conform to the space of the disk because the disk bulges maximally in the center. The disk space is wider in the middle and narrower at its anterior and posterior ends. For this reason, the various bone plugs which are currently available commercially have only four contact points, i.e. at the front and back of the disk space. Secondly, access to the disk is from the side of the dorsal spine of the adjacent vertebrae, leaving a space that is "off-center" relative to the bodies of the adjacent vertebrae such that the stability of the implant is even more problematical than might be apparent from the limited contact resulting from the shape of the intervertebral space. Another complication is the possibility of infection or other conditions which may require the removal of the implant. Also, if the bone pieces do not fuse, they may eventually extrude out of the disk space, causing pressure on the nerve roots.

Various prosthetic disk plugs, or implants, are disclosed in the art, but all are characterized by limitations of not conforming to the shape of the disk space, lack of stability when inserted off-center, inability to be removed, or other disadvantages. For instance, U.S. Patent No. 4,863,476 (and its European counterpart, EP-A-0260044) describes an elongated body divided longitudinally into two portions having a cam device movable therebetween for increasing the space between the two body portions once inserted into the disk space. However, that device is generally cylindrical in shape such that the only contact points between the device and the vertebral bodies are at the front and back of the disk space, creating increased likelihood of instability and generally rendering that device unsuitable for use after partial diskectomy. Cylindrically-shaped implants are also shown in DE-A-3 505 567 and EP-A-0 307 241 which are characterized by this same instability.

The prosthesis shown in EP-A-0 042 271 provides convex upper and lower surfaces and therefore addresses the concern as to instability. However, the generally rectangular shape of that implant precludes the possibility of rotating the implant on screw threads for precise positioning which would maximize the benefit on the convex shape of that implant. Instead, that implant is also wedge-shaped and is provided with anchoring grooves rather than helical screw threads and is designed to be tapped into place with a hammer, not rotated or screwed into place. One end of the implant is even provided with a flange to prevent penetration to an excessive depth into the spinal joint when hammered into place.

The art also discloses intervertebral disk prostheses (e.g., U.S. Patent Nos. 3,867,728, 4,309,777, 4,863,477 and 4,932,969 and French Patent Application No. 8816184) which may have more general contact with the adjacent disks than those implants which are cylindrically-shaped, but which are not intended for use in fusion of the disks. The art also includes spinal joint prostheses such as is described in U.S. Patent No. 4,759,769, which is again not indicated for use when fusion is the preferred surgical intervention.

There is, therefore, a need for a device capable of stabilizing the vertebrae adjacent an intervertebral disk, but which is also removable, for use in spinal fusion.

These needs are met in the present invention by providing an intervertebral disk stabilizing implant having a generally cylindrical, elongate shape and having end and middle portions, the middle portion having a cross-sectional area larger than that of the end portions characterized in that the cross-sectional area along the length of the implant is circular or elliptical and in that screw threads are formed on at least a forward portion of the outside surface of said implant and means are provided at one end of said implant suitable for detachably mounting an applicator.

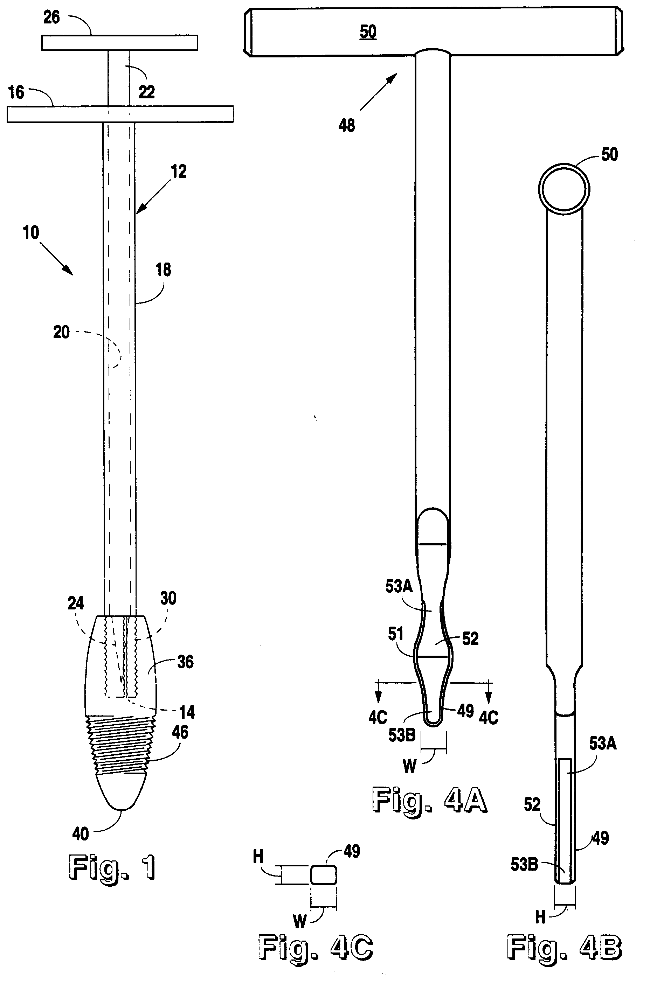

The present invention also provides a kit including the stabilizing implant in combination with a spreader for insertion into the disk space between adjacent vertebrae from the side of the dorsal spine after laminatomy to facilitate insertion of said stabilizing implant in an anterior-posterior direction, the spreader being comprised of a handle having an implant portion fixedly mounted thereto.
Referring now to the figures, Figure 1 is a plan view of a preferred embodiment of a vertebral disk stabilizing implant constructed in accordance with the present invention, attached to an applicator.
Figure 2 is a projected view of the applicator shown in Fig. 1 after being detached from the implant.
Figure 3 is a projected view of the stabilizing implant of Fig. 1 after being detached from the applicator.
Figure 4A, 4B, and 4C are plan, side, and cross-sectional views, respectively (Fig. 4C being taken along the lines 4C-4C in Fig. 4A), of a spreader which is part of a kit used to facilitate the insertion of the implant Fig. 3 between two adjacent vertebrae of a patient's spinal column.
Figure 5 is a plan view of a second embodiment of a spreader which is part of a kit in accordance with the present invention.
Figure 6 is a lateral view of a portion of a human spinal column having the stabilizing implant of Fig. 3 inserted therein and having a portion of the bodies of the vertebrae adjacent the implant shown cut away to show the engagement of the vertebral bodies by the implant.
Figures 7A is a longitudinal sectional view of an applicator for a middle-expandable stabilizing implant, not part of the present invention, shown in Figures 7B and 8.
Figures 7B and 8 are a longitudinal section and a plan view of a stabilizing implant after being detached from the applicator shown in Figure 7A.
Figure 9 is a plan view of another embodiment of a stabilizing implant constructed in accordance with the present invention, attached to an applicator.
Figure 10 is a projected view of the applicator shown in Figure 9 after detaching the implant therefrom.
Figure 11 is a projected view of the stabilizing implant of Fig. 9 after being detached from the applicator.
Figure 12 is a longitudinal, sectional view of a portion of the stabilizing implant of Fig. 9, taken along the lines 12-12 in Fig. 9.
Figure 13 is a projected view of another embodiment of a stabilizing implant constructed in accordance with the present invention and which is used in place of the implant of Fig. 3.
Figure 14 is an exploded, projected view of another embodiment of a stabilizing implant constructed in accordance with the present invention, and a suitable applicator.
Figure 15 is a plan view of a third embodiment of a spreader which is part of a kit used to facilitate the insertion of the implant of Fig. 14 between two adjacent vertebrae of a patient's spinal column.

A first embodiment of the stabilizing implant of the present invention is described in more detail by referring to Fig. 1, showing an applicator 12 and an implant 14. Applicator 12 is shown with a handle 16 in the shape of a "T", but the handle 16 may take the form of any convenient hand grip or other structure which facilitates subsequent rotation of the implant 14 once inserted into the space between two adjacent vertebrae as more particularly described below.

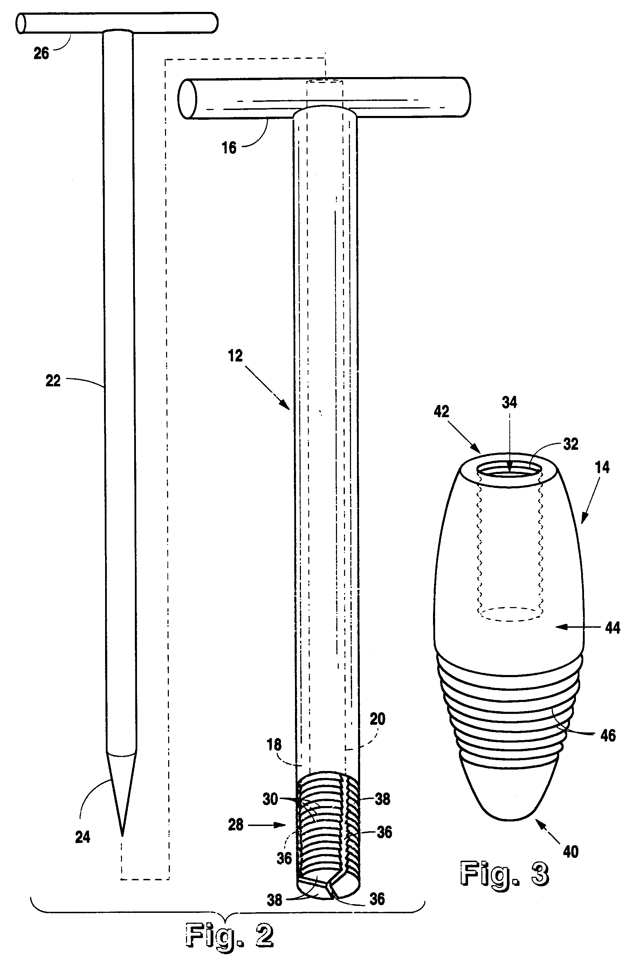

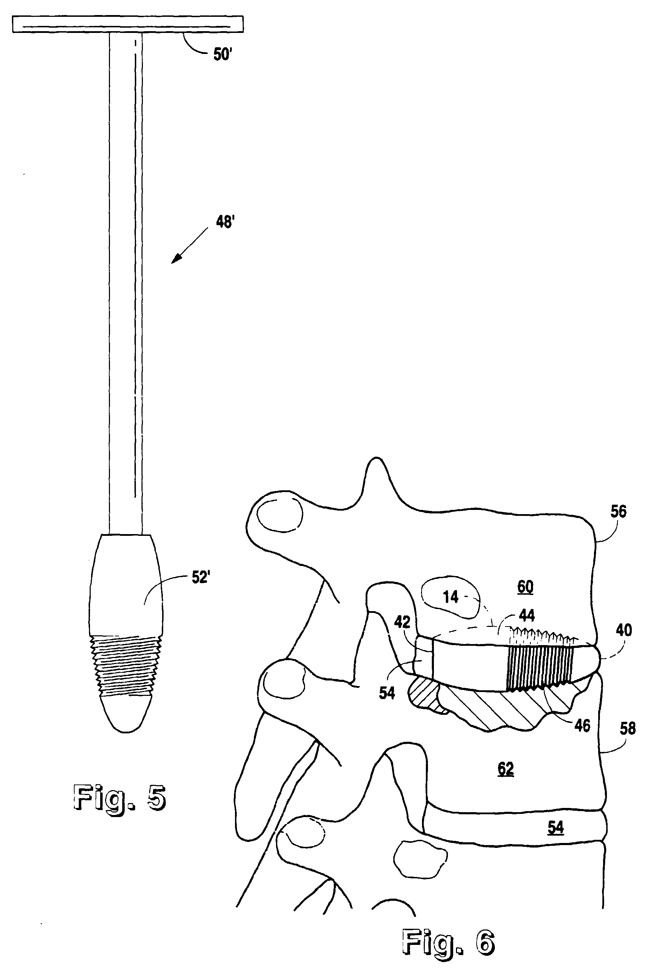

As shown more clearly when Fig. 1 is viewed in conjunction with Figs. 2 and 3, applicator 12 is comprised of an elongate shaft 18 having a longitudinal bore 20 therethrough, the bore 20 terminating in a point, with an elongate piston 22 disposed therein. Piston 22 is provided with a wedge-shaped, or pointed, end 24 which is sized to approximate the shape of the pointed end of the bore 20 in shaft 18 at one end and a handle 26 formed at the other end. As best shown in Fig. 2, the end 28 of shaft 18 is provided with screw threads 30 which mate with the threads 32 (see Fig. 3) formed in the interior wall of the bore 34 in implant 14. In this manner, the end 28 of shaft 18 is received in and positively engages the implant 14 to detachably mount implant 14 to applicator 12. Applicator 12 is provided with means for preventing relative rotational movement between the implant 14 and applicator 12 comprised, in the embodiment shown, of a plurality of radially spaced, longitudinal slots 36 in the end 28 of shaft 18 communicating with the longitudinal bore 20 therethrough and the wedge-shaped end 24 of piston 22. When piston 22 is forced down into the bore 20 in shaft 18, the pointed end 24 of piston 22 acts to spread, or force the portions 38 of the threaded end of shaft 18 between slots 36 outwardly into increasingly tighter frictional engagement with the interior wall of the bore 34 of implant 14, thereby preventing relative rotational movement of the implant 14 and applicator 12.

Implant 14 is formed in the shape of a generally elongate cylinder with a blunt, or rounded forward end 40 and rear end 42 having the aforementioned bore 34 opening therein. The diameter of the ends 40 and 42 is smaller than the diameter of the middle portion 44 of the implant for a purpose to be explained below. The outside surface of implant 14 is provided with threads 46, the function of which is also set out below. Implant 14 is preferably constructed of any durable, relatively biologically inert substance such as carbon fiber, titanium, several medical grade hard plastics, and such other materials as are known in the art for use in such implants.

Referring now to Figs. 4 and 5, there is shown a spreader, indicated generally at reference numeral 48, having integral handle 50 and implant portions 52. As shown in Figs. 4A, 4B, and 4C, although the shaft of the handle 50 of spreader 48 is substantially round in cross section and the elongate implant portion 52 is mounted on the end thereof, the cross-sectional shape of the implant portion 52 is substantially rectangular. In other words, opposed sides of the handle 50 are flattened at the end to which the implant portion 52 of spreader 48 is mounted. In this manner, the dimension of the implant portion 52 which represents the height (indicated at H on Figs. 4B and 4C) of the rectangular cross-section is minimized and the dimension of the implant portion 52 which represents the width (indicated at W on Figs. 4A and 4C) of the rectangular cross-section is maximized. The minimal dimension H facilitates insertion of the implant portion 52 into the disk space between two adjacent vertebrae through the small anterior-posterior opening between vertebrae and at the side of the dorsal spine which results from a laminotomy. After insertion of the implant portion 52 through that opening, the implant portion 52 is rotated by rotation of handle 50 so that the maximal dimension W of the implant portion 52 causes maximal spreading, or distraction, of the adjacent vertebrae. As best shown in Fig. 4C, the portions 49 of the implant portion 52 of spreader 48 which form the corners of the substantially rectangular cross-section thereof are rounded for reducing the resistance to rotation of the implant portion 52. The rounded corners 49 therefore effectively act as ramps conferring mechanical advantage on the implant portion 52 when rotated.

By reference to Fig. 4A, it can also be seen that the width of the maximal dimension W of the implant portion 52 of spreader 48 is greater in the middle portion 51 of the implant portion 52 than at the ends 53A and 53B of the implant portion 52. Further, the width of the maximal dimension W is greater at one end 53A of implant portion 52 than at the other end 53B, the end 53A being mounted to handle 50. This shape of implant portion 52 further reduces the resistance to rotation, distributes the force exerted against the bodies of the adjacent vertebrae that results from rotation over more surface area of the bodies of the adjacent vertebrae, and helps retain the implant portion 52 therebetween until it is desired to remove it from the disk space. The end 53B of implant portion 52 which is the end inserted first into the disk space is rounded to facilitate insertion and reduce the likelihood and severity of unintended trauma during insertion. The smaller maximum dimension W at the end 53B functions in similar fashion and the slope between the end 53B and middle portion 51' further facilitates introduction of structure having the maximal dimension W into the disk space.

A second embodiment of the spreader of the present invention is indicated generally at reference numeral 48' in Fig. 5. This second embodiment 48' is provided with a handle 50' and an implant portion 52' that is sized and generally cylindrically-shaped in the same size and dimensions as the stabilizing implant 14 and is used in the following manner.

The use of the stabilizing implant 14 of the present invention in, for instance, a method of lumbar interverbral disk stabilization, or "LIDS", is illustrated in Fig. 6. Surgery is performed as in a simple diskectomy and the intervertebral disk 54 is exposed through a small laminotomy. The disk material is removed and any nerve root compression is corrected. The posterior longitudinal ligament and disk cartilage are removed until the surfaces of the bodies 60 and 62 of adjacent vertebrae 56 and 58, respectively, are exposed above and below the disk space.

Using either of the spreaders 48 or 48', the vertebrae 56 and 58 are distracted to open the disk space, and once the desired "spread" has been achieved, the middle portion of the disk space is packed with cancellous bone chips (not shown). As described below, a kit of several spreaders, each having progressively larger diameter implant portions, is used to achieve the desired spread. Because the posterior longitudinal ligament is left intact to the opposite side and to the center of the disk space, the bone chips are held in place in the disk space. The appropriately-sized implant 14 is then inserted into the disk space using the applicator 12 until the threads 46 formed on the outside surface of implant 14 engage the bodies 60 and 62 of the adjacent vertebrae 56 and 58, respectively. Piston 22 is then wedged into the bore 20 to cause the applicator 12 to frictionally engage implant 14 to prevent relative rotational movement therebetween and the stabilizer 10 is rotated. Rotation of the implant 14 in the disk space causes the threads 46 to bear against the bodies 60 and 62 to move the implant further into (or back out of, depending upon the direction of rotation) the disk space in an anterior-posterior direction so as to enable the implant 14 to be positioned in the disk space at a position in which the expanded, or larger diameter portion 44 and the smaller diameter ends 40 and 42 of implant 14 contact the respective lower and upper surfaces of the bodies 60 and 62 of the adjacent vertebrae 56 and 58. The respective lower and upper surfaces of the vertebral bodies 60 and 62 are slightly concave such that the expanded middle portion 44 of implant 14 allows the implant 14 to engage substantially more of the respective surfaces of the vertebral bodies 60 and 62 than conventional prosthetic devices, thereby providing increased stability to the fusion.

Once positioned in the disk space so as to provide maximum stabilization, pressure on the piston 22 is released and the piston 22 is backed out of the bore 34 so as to allow the applicator 12 to be rotated without rotating the implant 14. The applicator is then detached from the implant 14 by unscrewing and backed out of the incision. If necessary, a small amount of physiologically compatible adhesive is applied over the cancellous bone chips just medial to the implant to close off the remaining portion of the opening into the disk space. The patient should be able to ambulate soon after the LIDS procedure because of the stability imparted to the spinal column by the implant of the present invention. Before narrowing of the disk space occurs, the cancellous bone chips will have started the fusion process.

The stabilizing implant is also used to advantage to perform, for instance, a posterior lateral intertransverse fusion. The implant 14 is inserted into the region of the disk space from which a portion of the disk has been removed as described above and the posterior lateral fusion performed. Because the implant 14 provides stability to the spine until the posterior lateral fusion is solid, the patient is generally able to ambulate soon after surgery. This procedure also prevents the narrowing of the disk space, which is a common problem with posterior lateral fusion.

Removal of the implant 14 is accomplished with relative ease compared to conventional implants. The shaft 18 of applicator 12 is screwed back into the threaded bore 34 in implant 14, piston 22 is re-inserted into the longitudinal bore 20 in applicator 12, and by applying pressure to piston 22 to prevent relative rotation between implant 14 and applicator 12, the implant is rotated to cause posteriorly-directed movement of the implant 14 out of the disk space.

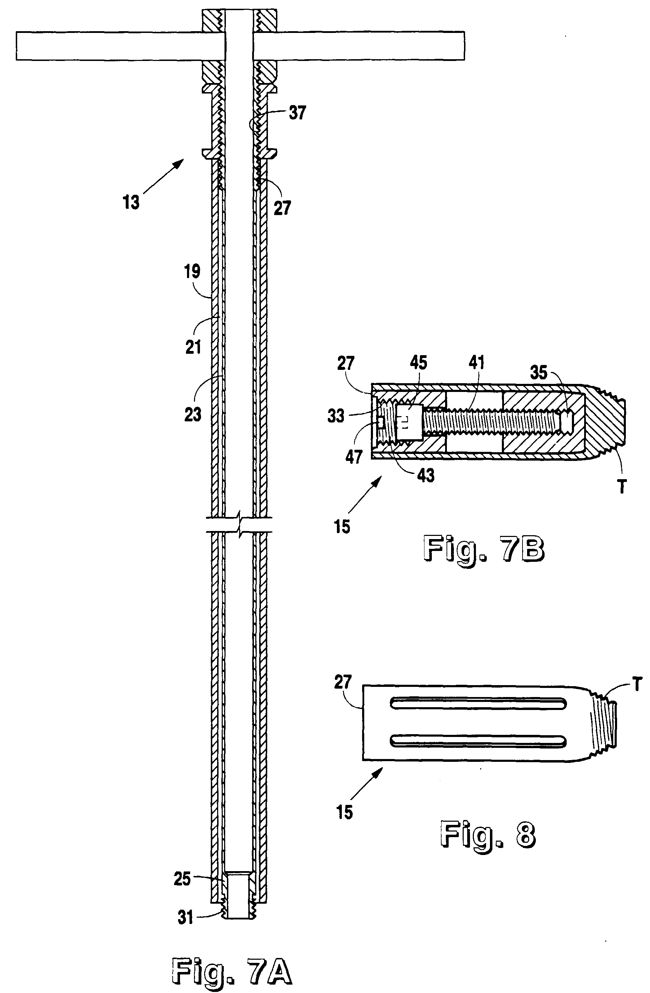

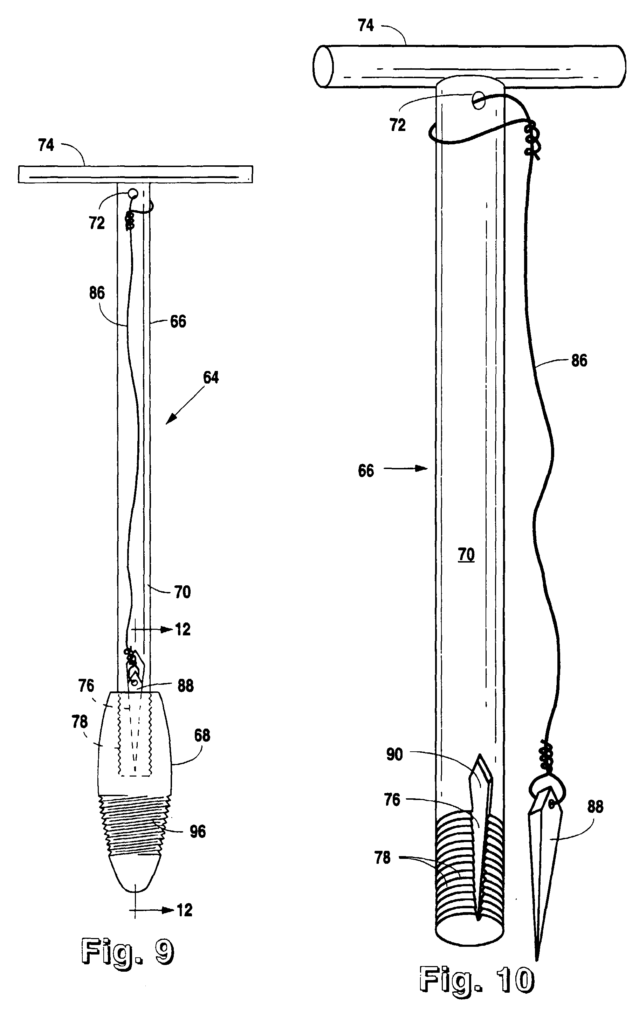

Referring now to Figs. 7A, 7B, and 8, which show an applicator 13 (Fig. 7A) and an implant 15 (Fig. 7B), not part of the present invention, but described in my International Patent Application PCT/US92/01397 (WO 92/14423); the implant 15 being mounted on the end of applicator 13 as will be described. Applicator 13 is comprised of an elongate shaft 19 having a longitudinal bore 21 therethrough with an elongate mandrel 23 disposed therein. Mandrel 23 is provided with an end 25 having screw threads 31 formed thereon which mate with the screw threads 33 (see Fig. 7B) formed in the interior wall of the bore 35 in implant 15. In this manner, the end 25 of mandrel 23 is received in and affirmatively engages the implant 15 to detachably mount implant 15 to applicator 13.

Implant 15 is detached from the applicator 13 by rotating mandrel 23, causing the mandrel 23 to back out of bore 21 as a result of the engagement of the threads 29 formed thereon with the threads 37 formed in the wall of the bore 21 in applicator 13, until the end 27 of the implant 15 contacts the end 39 of applicator 13. The friction resulting from that contact prevents further rotation of implant 15 and as a result of the continued rotation of mandrel 23, the implant 15 is detached therefrom.

Referring to Figs. 7B and 8, the implant 15 is shown in detail. Implant 15 is constructed and functions as described in my International Application No. PCT/US92/01397 (WO 92/14423) and so is described only briefly here. The implant 15 is formed in the shape of a generally elongate, hollow cylinder comprised of a flexible plastic, metal, or similar medically inert material of the type described above with a screw 41 positioned in the threaded bore 35 therethrough. Also positioned in the bore 35 is a jam nut 43 which is formed in the shape of a ring to allow access to the screwdriver slot formed in the head 45 of screw 41 and which is itself provided with a slot 47 for receiving a screwdriver (not shown). Upon rotation of screw 41, the ends of implant 15 are drawn towards each other, causing expansion of the middle portion thereof. Referring again to Fig. 7A, it can be seen that the mandrel 23 positioned in applicator 23 is provided with a bore B for receipt of a screwdriver therethrough to facilitate rotation of screw 41. Once sufficient expansion is achieved, a second screwdriver replaces the first for rotation of jam nut 43 against the head 45 of screw 41, thereby preventing further rotation. The end of implant 15 is rounded and provided with threads T in the same manner and for the same purposes as implant 14. Once the jam nut 43 is tightened against the head 45 of screw 41, the screwdriver slot 47 in jam nut 43 and the slot in the head 45 operate in conjunction with the threads T on the outer surface of implant 15 to allow fine positioning of the implant 15 in the disk space.

Referring now to Figs. 9-12, another embodiment of the stabilizing implant of the present invention is indicated generally at reference numeral 68. Applicator 66 and implant 68 have the same general function and component parts as those shown in Figs. 1 to 3. However, the shaft 70 of applicator 66, rather than being provided with a bore having a piston disposed therein as with the bore 20 and piston 22 of the applicator 12, is provided with a hole 72 near the end in which the handle 74 is formed and a longitudinal groove 76 crossing the threads 78 formed in the end of shaft 70 which is received by the threads 80 in the bore 82 of implant 68. A similar groove 84, best shown in Fig. 9, runs longitudinally across the threads 80 in the bore 82 of implant 68. A safety line, or wire, 86 is threaded through the hole 72 in shaft 70 having a wedge-shaped key 88 attached to the other end thereof and, when the implant 68 is mounted to the shaft 70 of applicator 66 and the grooves 76 and aligned, key 88 is wedged into the key slot formed by the aligned grooves 76 and 84 by insertion into the portion 90 of the groove 76 formed at the end of the shaft 70 which extends beyond the smaller diameter end portion 92 (compared to the diameter of the expanded middle portion 94) of implant 68, the applicator 66 and implant 68 are locked up to prevent relative rotation therebetween so that the threads 96 formed on the external surface of implant 68 function in the same manner and for the same purpose as described above with reference to Figures 1-3.

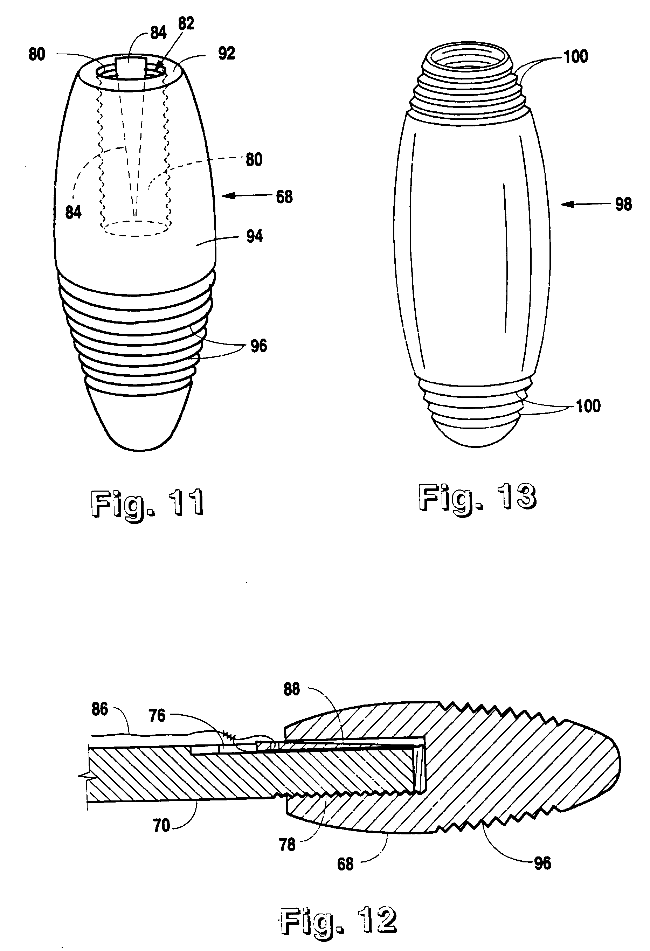

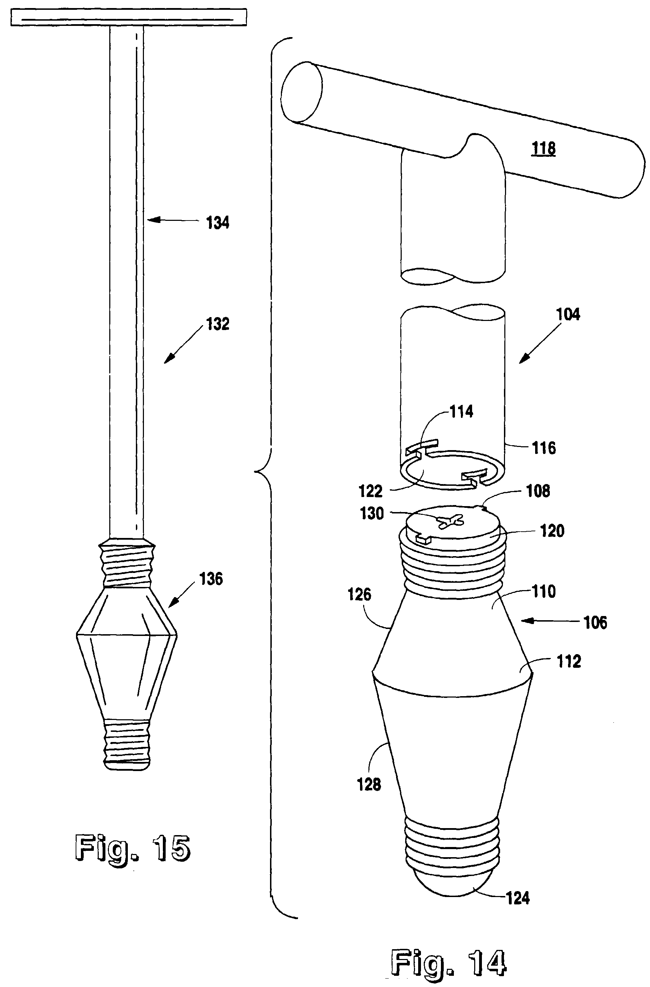

In certain applications, for instance, when fusion is being performed on a patient having deteriorating vertebrae, it may be desirable to have threads formed at more than one location on the external surface of the implant. In such circumstances, an implant such as the implant indicated at reference numeral 98 in Fig. 13 is utilized in connection with .the applicator 12 shown in Figs. 1-3 (implant 98 may also be provided with a groove such as the groove 84 in the implant 68 of Figs. 9-12 for use in connection with the applicator 66). Implant 98 is provided with two sets of threads 100 formed on the external surface thereof to increase the likelihood that the threads 100 will bear against the bodies of the vertebrae between which it is inserted to facilitate the anterior-posterior positioning of the implant 98 in the anatomical region of the disk space.

Referring now to Fig. 14, there is shown yet another embodiment of a disk stabilizing implant, indicated generally at reference numeral 106, constructed in accordance with the present invention. Implant 106 is detachably mounted to an applicator 104 by receipt of the keys 108 formed in the smaller diameter end portion 110 (compared to the expanded middle portion 112) of implant 106 by the T-slots 114 formed at the end of the shaft 116 of applicator 104 opposite handle 118. To provide additional rigidity to the mounting of implant 106 to applicator 104, the end 110 of implant 106 is provided with a cylindrical extension 120 which is received within a similarly dimensioned cavity 122 formed on the end of shaft 116, the external surface of extension 120 bearing against the inside wall of the cavity 122 to stabilize the implant 106 on the end of applicator 104.

Implant 106 is, like each of the implants 14, 68, and 98, formed in the shape of a generally elongate cylinder having end 110 and 124 and middle 112 portions, the diameter of the middle portion 112 being larger than the diameter of the end portions 110 and 124. In the case of the implant 106, however both ends 110 and 124 are provided with threads on the external surface of the implant and the larger diameter middle portion 112 is not located in equidistant from the ends 110 and 124. Instead, the largest diameter of the implant 106 is located closer to the end 110 of the implant 106 which is located posteriorly when inserted into the disk space such that the slope of the external surface 126 between the largest diameter of the middle portion 112 and end 110 is greater than the slope of the external surface 128 of implant 106 between the middle portion 112 and end 124. By shaping implant 106 in this manner, the increased slope of the surface 126 helps to prevent undesirable posterior movement of the implant 106 in the disk space once inserted. To decrease any tendency of the implant 106 to move in the anterior direction, the diameter of the end portion 124 of implant 106 is optionally larger than the diameter of the end portion 110. As is the case with each of the implants 14, 68, and 98, the end 124 of implant 106 is formed in a blunt, or rounded, shape to reduce the likelihood of injury to the nerves of the spinal cord during insertion into the disk space. To further facilitate proper anterior-posterior positioning of the implant 106 in performing the above-described LIDS procedure, the surface of the extension 120 at the end 110 of implant 106 is provided with a slot 130 for receiving a screwdriver blade (not shown) for fine adjustment of the position in the disk space.

A spreader, indicated generally at reference numeral 132 in Fig. 15, is provided for spreading the adjacent vertebrae for insertion of the implant 106. Spreader 132 is formed of an integral handle 134 and implant portion 136, the latter being formed in the same approximate shape as the implant 106 of stabilizer 102.

It is advantageous to provide a kit comprised of a number of spreaders 48, 48' or 132, depending upon the particular stabilizing implant being employed, of progressively larger diameters to obtain the desired degree of spread of the vertebrae adjacent the disk space into which the stabilizing implant 14, 15, 68, 98, or 106 is to be inserted. The kit of spreaders are of increasingly larger diameters (for instance, 6, 8, 10, and 12 mm or, in the case of spreader 48, it is the maximal dimension W which is sized to these dimensions) in their respective expanded middle portions; it is also advantageous to supply spreaders in the kit having implant portions of different lengths. Likewise, it is advantageous to include implants in the kit of different diameters and lengths to obtain the best fit between the anatomical region of the disk space into which the implant is being inserted and the shape of the implant so as to be able to position the implant in the disk space at which the largest proportion of the external surface of the implant bears against the surfaces of the bodies of the adjacent vertebrae, thereby maximizing the stabilizing properties of the implant.

Although described in terms of the several embodiments shown in the figures, those embodiments are shown to exemplify the present invention, it being recognized that certain changes can be made to the specific structure of these various embodiments shown and described without departing from the present invention. For instance, there are many ways other than those illustrated to mount the implant to the applicator so as to selectively prevent relative rotation therebetween while still enabling the applicator to be detached from the implant once positioned in the disk space. Likewise, the implant is described herein as being "generally cylindrical" in shape, but additional stability may be obtained by forming the implant with a slightly ovoid, or elliptical, cross-sectional shape while retaining the elongate, generally cylindrical shape of the implant. In the case of an implant such as is contemplated in Fig. 3 but having a "flattened" cross-sectional shape, it will be recognized that the grooves 76 and 84 in the applicator 66 and implant 68, respectively, are aligned with both the handle 74 of applicator 66 and either the minimum or the maximum dimension of the cross-sectional shape of the implant to facilitate rotation of the implant in the disk space so as to enable the maximum surface area of the implant (e.g., the flattened" surface) to bear against the bodies of the respective adjacent vertebrae.

## Claims

1. An intervertebral disk stabilizing implant (14, 68, 98, 106) having generally cylindrical, elongate shape and having end (40, 42) and middle (44) portions, the middle portion having a cross-sectional area larger than that of the end portions (40, 42), characterized in that the cross-sectional area along the length of the implant is circular or elliptical and in that screw threads (46) are formed on at least a forward portion of the outside surface of said implant (14) and means (32, 34) are provided at one end (42) of said implant (14) suitable for detachably mounting an applicator.

2. A kit including the stabilizing implant of claim 1, in combination with a spreader (48, 48'; 132) for insertion into the disk space between adjacent vertebrae from the side of the dorsal spine after laminatomy to facilitate insertion of said stabilizing implant (14, 68, 98, 106) in an anterior-posterior direction, the spreader being comprised of a handle (50, 50', 134) implant portion (52, 52', 136) fixedly mounted thereto.

3. The kit of claim 2, wherein the implant portion (52, 52', 136) of said spreader is shaped and sized in approximately the same size and shape as the stabilizing implant (14, 68, 98, 106).

4. The kit of claim 2, additionally comprising a plurality of spreaders having implant portions of progressively larger diameters.

5. The kit of claim 2, wherein said implant portion (52) of the spreader (48) has a substantially rectangular cross-sectional shape, the rectangular cross-sectional shape being minimized in height (H) to facilitate insertion of said implant portion into the disk space and maximized in width (W) for rotation by approximately 90° by rotation of said handle after insertion into the disk space to spread the vertebrae adjacent the disk space apart from each other.

6. The kit of claim 5, wherein the corners (49) of the substantially rectangular cross-sectional shape of said implant portion (52) of the spreader (48) are rounded to facilitate rotation of said spreader after insertion of said implant portion thereof into the disk space.

7. The kit of claim 5, wherein the width of said implant portion (52) of the spreader (48) is greater in the middle portion (51) than at the ends (53A, 53B) thereof.

8. The kit of claim 7, wherein the width of said implant portion (52) of the spreader (48) is greater at one end (53A) thereof than at the other (53B).

9. The kit of claim 8, wherein said implant portion (52) of the spreader (48) is mounted to said handle at the end (53A) of said implant portion having the greater width.

10. A kit including a plurality of the spreaders of any of claims 5-9, each of said plurality of spreaders having an implant portion of progressively larger width, for insertion into and removal from the disk space in sequential fashion until the adjacent vertebrae are spread to a desired degree.

## Patentansprüche

1. Implantat (14, 68, 98, 106) zur Stabilisierung von Bandscheiben mit einer im allgemeinen zylindrischen, gestreckten Form und Endabschnitten (40, 42) und einem Mittelabschnitt (44), wobei der Mittelabschnitt eine Querschnittsfläche aufweist, die größer als die der Endabschnitte (40, 42) ist, dadurch **gekennzeichnet**, daß die Querschnittsfläche entlang der Länge des Implantats kreisförmig oder elliptisch ausgebildet ist, daß aufwenigstens einem Vorderabschnitt der äußeren Oberfläche des Implantats (14) Schraubengewinde (46) ausgebildet sind, und daß an einem Ende (42) des Implantats (14) Mittel (32, 34) vorgesehen sind, die für die lösbare Montage eines Anwenders geeignet sind.

2. Bausatz, der das Stabilisierungsimplantat nach Anspruch 1 zusammen mit einem Spreizbauteil (48, 48'; 132) zum Einbringen in dem Scheibenraum zwischen benachbarten Bandscheiben von der Seite des Dorsal-Rückenmarks nach einer Schichtentfernung umfaßt, um das Einbringen des Stabilisierungsimplantats (14, 68, 98, 106) in einer Vorder-Hinter-Richtung zu vereinfachen, wobei das Spreizbauteil einen Griffabschnitt (50, 50', 134) und einen Implantatabschnitt (52, 52'; 136) aufweist, die an dem Spreizbauteil fest montiert sind.

3. Bausatz nach Anspruch 2, wobei der Implantatabschnitt (52, 52', 136) des Spreizbauteils etwa dieselbe Größe und dieselbe Form wie das Stabilisierungsimplantat aufweist (14, 68, 98, 106).

4. Bausatz nach Anspruch 2, **gekennzeichnet** durch mehrere Spreizbauteile, die Implantatabschnitte mit zunehmend größeren Durchmessern aufweisen.

5. Bausatz nach Anspruch 2, wobei der Implantatabschnitt (52) des Spreizbauteils (48) eine im wesentlichen rechteckige Querschnittsform aufweist, wobei die rechteckige Querschnittsform in der Höhe (H) minimiert ist, um das Einbringen des Implantatabschnitts in dem Scheibenraum zu erleichtern, und wobei die rechteckige Querschnittsform in der Breite (B) für die Drehung um etwa 90° mit Hilfe des Drehens des Griffs nach dem Einbringen in dem Scheibenraum maximiert ist, um die Bandscheiben auseinander zu spreizen, die benachbart zu dem Scheibenraum angeordnet sind.

6. Bausatz nach Anspruch 5, wobei Ecken (49) der im wesentlichen rechteckigen Querschnittsform des Implantatabschnitts (52) des Spreizbauteils (48) abgerundet sind, um das Drehen des Spreizbauteils nach dem Einbringen des Implantatabschnitts in dem Scheibenraum zu erleichtern.

7. Bausatz nach Anspruch 5, wobei die Breite des Implantatabschnitts (52) des Spreizbauteils (48) in dem mittleren Abschnitt (51) größer als an den Enden (53A, 53B) ist.

8. Bausatz nach Anspruch 7, wobei die Breite des Implantatabschnitts (52) des Spreizbauteils (48) an einem Ende (53A) größer als an einem anderen Ende (53B) ist.

9. Bausatz nach Anspruch 8, wobei der Implantatabschnitt (52) des Spreizbauteils (48) am Griff an dem Ende (53A) des Implantatabschnitts montiert ist, das die größere Breite aufweist.

10. Bausatz mit mehreren Spreizbauteilen nach einem der Ansprüche 5 bis 9 zum aufeinanderfolgenden Einbringen in und Entfernen aus dem Scheibenraum, bis die benachbarten Bandscheiben in einem gewünschten Ausmaß auseinandergespreizt sind, wobei jedes der mehreren Spreizbauteile einen Implantatabschnitt mit zunehmend größerer Breite aufweist.

## Revendications

1. Implant de stabilisation de disque intervertébral (14, 68, 98, 106) ayant une forme allongée de manière générale cylindrique et ayant des parties d'extrémité (40, 42) et une partie médiane (44), la partie médiane ayant une surface en coupe plus grande que celle des parties d'extrémité (40, 42), caractérisé en ce que la surface en coupe le long de la longueur de l'implant est circulaire ou elliptique et en ce que qu'un pas de vis (46) est formé sur au moins une partie avant de la surface extérieure dudit implant (14) et des moyens (32, 34) sont prévus au niveau d'une extrémité (42) dudit implant (14), adaptés pour monter de manière amovible un applicateur.

2. Kit incluant l'implant de stabilisation de la revendication 1 en combinaison avec un écarteur (48, 48'; 132), pour être inséré dans un espace de disque situé entre des vertèbres adjacentes à partir du côté de la colonne vertébrale après laminectonie pour faciliter l'insertion dudit implant de stabilisation (14, 68, 98, 106) dans une direction antérieure-postérieure, l'écarteur étant constitué d'une poignée (50, 50', 134), une partie d'implant (52, 52', 136) étant montée de manière fixe sur celle-ci.

3. Kit selon la revendication 2, dans lequel la partie d'implant (52, 52', 136) dudit écarteur est mise en forme et dimensionnée approximativement selon la même forme et la même dimension que l'implant de stabilisation (14, 68, 98, 106).

4. Kit selon la revendication 2, comportant de plus plusieurs écarteurs ayant des parties d'implant de diamètre progressivement plus grand.

5. Kit selon la revendication 2, dans lequel ladite partie d'implant (52) de l'écarteur (48) a une forme en coupe sensiblement rectangulaire, la forme en coupe rectangulaire étant minimisée en hauteur (H) pour faciliter l'insertion de ladite partie d'implant dans l'espace de disque et maximisée en largeur (W) pour pouvoir tourner sur approximativement 90 degrés par mise en rotation de ladite poignée après insertion dans l'espace de disque pour écarter les vertèbres adjacentes à l'espace de disque.

6. Kit selon la revendication 5, dans lequel les coins (49) de la forme en coupe sensiblement rectangulaire de ladite partie d'implant (52) de l'écarteur (48) sont arrondis pour faciliter la mise en rotation dudit écarteur après insertion de ladite partie d'implant de celui-ci dans l'espace de disque.

7. Kit selon la revendication 5, dans lequel la largeur de ladite partie d'implant (52) de l'écarteur (48) est plus grande dans la partie médiane (51) qu'au niveau de ses extrémités (53A, 53B).

8. Kit selon la revendication 7, dans lequel la largeur de ladite partie d'implant (52) de l'écarteur (48) est plus grande au niveau d'une première extrémité (53A) qu'au niveau de l'autre (53B).

9. Kit selon la revendication 8, dans lequel la partie d'implant (52) de l'écarteur (48) est monté sur ladite poignée au niveau de l'extrémité (53A) de ladite partie d'implant ayant la largeur plus grande.

10. Kit comportant une pluralité d'écarteurs selon l'une quelconque des revendications 5 à 9, chacun de ladite pluralité d'écarteurs ayant une partie d'implant de largeur progressivement plus grande, destinée à être insérée dans l'espace disque et enlevée de l'espace disque de manière séquentielle jusqu'à ce que les vertèbres adjacentes soient écartées sur une amplitude voulue.
